(19) **Europäisches Patentamt**
**European Patent Office**
**Office européen des brevets**

(11) **EP 1 629 076 B1**

(12) **EUROPÄISCHE PATENTSCHRIFT**

(45) Veröffentlichungstag und Bekanntmachung des Hinweises auf die Patenterteilung:
**05.05.2010 Patentblatt 2010/18**

(21) Anmeldenummer: **04734247.2**

(22) Anmeldetag: **21.05.2004**

(51) Int Cl.:
*C11D 17/00* (2006.01)    *C11D 3/37* (2006.01)

(86) Internationale Anmeldenummer:
**PCT/EP2004/005517**

(87) Internationale Veröffentlichungsnummer:
**WO 2004/104158 (02.12.2004 Gazette 2004/49)**

(54) **EIN TENSID UND EIN COTENSID UMFASSENDE MISCHUNG**

MIXTURE COMPRISING A SURFACTANT AND A COSURFACTANT

MELANGE COMPRENANT UN TENSIO-ACTIF ET UN CO-TENSIO-ACTIF

(84) Benannte Vertragsstaaten:
**AT BE BG CH CY CZ DE DK EE ES FI FR GB GR HU IE IT LI LU MC NL PL PT RO SE SI SK TR**

(30) Priorität: **22.05.2003 DE 10323180**

(43) Veröffentlichungstag der Anmeldung:
**01.03.2006 Patentblatt 2006/09**

(73) Patentinhaber: **BASF SE**
**67056 Ludwigshafen (DE)**

(72) Erfinder:
• **MOCK-KNOBLAUCH, Cordula**
**67063 Ludwigshafen (DE)**
• **WAGNER, Norbert**
**67112 Mutterstadt (DE)**
• **OETTER, Günter**
**67227 Frankenthal (DE)**
• **VÖLKEL, Ludwig**
**67117 Limburgerhof (DE)**
• **PETROVIC, SUSANNE**
**67063 Ludwigshafen (DE)**
• **FECHTENKÖTTER, Andreas**
**67063 Ludwigshafen (DE)**
• **CHRISSTOFFELS, Lysander**
**67117 Limbergerhof (DE)**
• **KLÜGLEIN, Matthias**
**67071 Ludwigshafen (DE)**
• **BECKER, Stefan**
**68167 Mannheim (DE)**

(56) Entgegenhaltungen:
**EP-A- 0 794 245**

**Beschreibung**

Die Erfindung betrifft eine Mischung, enthaltend ein Tensid und ein Cotensid, eine Verwendung einer Mischung zum Stabilisieren von Emulsionen, eine Mikroemulsion, enthaltend ein Tensid und ein Cotensid, eine Verwendung einer Mischung oder einer Mikroemulsion sowie Wasch-, Reinigungs-, Desinfektions-, Netz-, Beschichtungs-, Klebe-, Lederentfettungs-, Feuchthalte- oder Textilbehandlungsmittel oder pharmazeutische, Lebensmittel-, Pflanzenschutz- oder kosmetische Formulierungen, insbesondere Sonnenschutz-, Hautpflege- oder Haarstylingmittel, Duschgele, Shampoos, Badezusätze oder Duftöle.

Tenside sind Substanzen, die die Grenzflächenspannung zwischen miteinander nicht mischbaren flüssigen Phasen, einer polaren Phase, häufig Wasser und einer unpolaren, organischen Phase herabsetzen und somit ihre gegenseitige Löslichkeit erhöhen. Tenside verfügen über einen charakteristischen Aufbau und weisen mindestens eine hydrophile und eine hydrophobe Struktureinheit auf. Dieser Aufbau wird auch als amphiphil bezeichnet.

Tenside sind ökologisch besonders relevante Stoffe, deren Umweltverträglichkeit sichergestellt werden muss. Neben einer guten Abbaubarkeit von Tensidrückständen in Abwässern ist es daher besonders wichtig, die eingesetzten Tensidmengen möglichst ohne Beeinträchtigung ihrer Wirksamkeit herabzusetzen, das heißt die Effizienz der Tenside zu steigern. Als Tensideffizienz wird dabei üblicherweise die Menge an Tensid bezeichnet, die benötigt wird, um einen bestimmten Effekt zu erzielen, zum Beispiel um den Anteil unpolarer Phase in der polaren Phase bzw. umgekehrt zu solubilisieren oder um die Oberflächenspannung bei möglichst niedriger Konzentration möglichst stark zu verringern.

Übliche konventionelle Emulsionen können Öl- und Wasserphase in sehr unterschiedlichen Volumenanteilen enthalten. Sie haben eine kontinuierliche und eine disperse Phase, die als sehr kleine, durch Belegung mit Tensiden stabilisierte Kügelchen in der kontinuierlichen Phase vorliegt. Je nach der Natur der kontinuierlichen Phase spricht man von Öl-in-Wasser- oder Wasser-in-Öl-Emulsionen. Diese Emulsionen sind im Idealfall kinetisch stabil, d. h. sie bleiben auch längere Zeit, aber nicht unbegrenzt, erhalten. Insbesondere bei Temperaturschwankungen können sie zur Phasentrennung durch Absitzen, Aufrahmen, Verdicken oder Flocken neigen.

Sogenannte Mikroemulsionen sind thermodynamisch stabile, fluide, optisch klare Formulierungen zweier nicht mischbarer Flüssigkeiten, wie Öl und Wasser. Mikroemulsionen entstehen, wenn ein Tensid, oder häufiger eine Mischung aus einem Tensid und einem Cotensid die Grenzflächenspannung Öl/Wasser auf extrem niedrige Werte häufig im Bereich $-10^{-3}$ bis $10^{-9}$, bevorzugt $10^{-4}$ bis $10^{-6}$ N/m absenkt, so dass durch die thermische Bewegung homogen die beiden unlöslichen Phasen allein dispergiert bleiben. Mikroemulsionen weisen häufig bikontinuierliche Strukturen auf mit Gleichgewichtsbereichen, sogenannten Sub-Phasen in der Größenordnung von 100 bis 1000 Angström (vgl. Advanced Materials, 2000, 12, Nr. 23, Seiten 1751 ff.).

Bikontinuierliche Mikroemulsionen enthalten zwei Phasen, eine Wasser- und eine Ölphase, in Form von ausgedehnten nebeneinanderliegenden und ineinander verschlungenen Domänen, an deren Grenzfläche stabilisierende grenzflächenaktive Tenside in einer monomolekularen Schicht angereichert sind. Bikontinuierliche Mikroemulsionen bilden sich sehr leicht, in der Regel wegen der sehr niedrigen Grenzflächenspannung spontan, wenn die Einzelkomponenten, Wasser, Öl und ein geeignetes grenzflächenaktives System, vermischt werden. Da die Domänen in mindestens einer Dimension nur sehr geringe Ausdehnungen in der Größenordnung von Nanometern haben, erscheinen die Mikroemulsionen visuell transparent und sind je nach dem eingesetzten grenzflächenaktiven System in einem bestimmten Temperaturbereich thermodynamisch, das heißt zeitlich unbegrenzt, stabil.

Bikontinuierliche Mikroemulsionen sind zum Beispiel in dem Artikel "Mikroemulsionen - eine wissenschaftliche und anwendungstechnische Fundgrube?" von H.-F. Eicke in SÖFW-Journal 118 (1992), Seiten 311 bis 314, beschrieben.

Zum Erreichen der erforderlichen niedrigen Grenzflächenspannung an den Phasengrenzen enthalten die Mikroemulsionen spezielle Amphiphile, das heißt grenzflächenaktive Mittel, und in ihrer wässrigen Phase häufig gelöste Elektrolyte und gegebenenfalls weitere Hilfsstoffe. Elektrolyte werden vor allem dann zugesetzt, wenn die Amphiphile zum Teil oder ausschließlich ionische Tenside sind.

Aus DE-A 198 39 054 ist es bekannt, die Effizienz von Tensiden durch Zugabe von Additiven zu steigern, wobei als Additive AB-Blockcopolymere mit einem wasserlöslichen Block A und einem wasserunlöslichen Block B eingesetzt werden. Die Blöcke A und B können dabei Molekulargewichte zwischen 500 und 60.000 g/mol annehmen. Als Block A wird bevorzugt ein Polyethylenoxid-Block eingesetzt, allgemein jedoch alle wasserlöslichen Blöcke, die in Verbindung mit Block B ein Amphiphil bilden. Für den Block B werden Polymerisate eines einzigen Monomers oder eines Monomerengemisches beschrieben.

Die beschriebenen Blockcopolymere haben jedoch insbesondere den Nachteil, dass sie nach Herstellungsverfahren erhältlich sind, die für den Labormaßstab, nicht jedoch für den großtechnischen Einsatz geeignet sind. Die genannte Druckschrift verweist zum Herstellungsverfahren auf die DE-A 196 34 477, worin die Polymerisation unter Verwendung von Alkalimetallorganylen; das heißt eine für den großtechnischen Einsatz ungeeignete Herstellungsmethode beschrieben wird.

Aus EP-A 0 794 245 sind Tenside mit polymeren kammartigen Strukturen bekannt, diese weisen jedoch ohne Ausnahme mindestens ein Heteroatom (Stickstoff oder Sauerstoff) in der das Rückgrat bildenden Kette auf.

Es war demgegenüber Aufgabe der Erfindung, Substanzen zur Verfügung zu stellen, die als Cotenside zur Steigerung der Effizienz von Tensiden in Emulsionen, insbesondere in Mikroemulsionen, einsetzbar sind, und die in wirtschaftlich vorteilhafter Weise, auf der Basis von großtechnischen Ausgangssubstanzen sowie auf großtechnisch realisierbaren Umsetzungswegen erhalten werden können.

Die Aufgabe wird durch eine Mischung, umfassend ein Tensid und ein Cotensid gelöst, die dadurch gekennzeichnet ist, dass man als Cotensid ein amphiphiles Kammpolymer einsetzt, aufweisend ein Rückgrat mit am Rückgrat angebrachten zwei oder mehreren Seitenketten, wobei sich die Seitenketten untereinander und/oder die Seitenketten vom Rückgrat in ihrem amphiphilen Charakter unterscheiden.

Es wurde überraschend gefunden, dass Cotenside, die die Struktur von Kammpolymeren aufweisen, für den erfindungsgemäßen Einsatz besonders geeignet sind.

Als Unterschied im amphiphilen Charakter wird vorliegend verstanden, dass sich die Seitenketten untereinander und/oder vom Rückgrat bezüglich ihrer Affinität zu polaren bzw. zu unpolaren Phasen unterscheiden.

Das Kammpolymer enthält bevorzugt sich wiederholende, jeweils eine oder mehrere Struktureinheiten A, A' und X, wobei die Struktureinheiten A und A' das Rückrat bilden und die Struktureinheiten A' eine Ankerfunktion zum Anbinden der die Seitenketten bildenden Struktureinheiten X haben, und wobei die Struktureinheiten A in einer Molfraktion n, die Struktureinheiten A' in einer Molfraktion m und die Struktureinheiten X in einer Molfraktion 1 vorliegen mit

$$n + m + 1 = 1,$$

$$n \geq m$$

und

$$1 > m \text{ oder } 1 = 0.$$

Das Kammpolymer kann vorzugsweise sowohl hydrophile als auch hydrophobe Seitenketten aufweisen.

In einer Ausführungsvariante hat das Rückgrat des Kammpolymers hydrophilen und alle Seitenketten des Kammpolymers hydrophoben Charakter. Besonders günstig ist es, wenn das im Rückgrat enthaltene Monomer A selber eine Seitenkette trägt, die sich in ihrem amphiphilen Charakter vorzugsweise von den an A' angebrachten Seitenketten unterscheidet.

Das Kammpolymer ist somit ein Copolymer, wobei die das Rückgrat bildenden, jeweils eine oder mehrere Struktureinheiten A und A' beliebig angeordnet sein können, das heißt sowohl streng alternierend (dann sind die Variablen n und m gleich) als auch als Blockcopolymere, als statistische Copolymere oder mit Gradienten.

Geeignet sind beispielsweise Copolymere, wie sie in EP-A 0 412 389 zur Verwendung als Mittel zum Hydrophobieren von Leder oder Pelzfellen beschrieben sind und durch radikalische Copolymerisation von $C_8$ bis $C_{40}$-Monoolefinen mit ethylenisch ungesättigten $C_4$-bis $C_8$-Dicarbonsäureanhydriden nach Art einer Substanzpolymerisation bei Temperaturen von 80 bis 300°C zu Copolymerisaten mit Molmassen von 500 bis 20.000 g/mol, anschließender Solvolyse der Anhydridgruppen der Copolymerisate und zumindest partielle Neutralisation der bei der Solvolyse entstehenden Carboxylgruppen in wässrigem Medium mit Basen erhältlich sind.

Bevorzugt haben die erfindungsgemäßen Cotenside Molekularmassen im Bereich von 500 bis 100.000 g/mol, besonders bevorzugt im Bereich von 1.000 bis 50.000 g/mol.

Vorzugsweise betragen die Molfraktionen n, m und 1 unabhängig voneinander 0,001 bis 99,9 %, bevorzugt ist n größer oder gleich m und 1 größer m oder 1 gleich Null. Besonders bevorzugt ist m zwischen 0,001 bis 0,5, n zwischen 0,001 bis 0,99 und n zwischen 0,2 und 0,99.

Vorteilhaft können zur Bildung der Struktureinheit(en) A Monomere eingesetzt werden, die eine oder mehrere hydrophobe Seitenketten tragen.

Die Struktureinheit oder Struktureinheiten A sind vorteilhaft aus einem oder mehreren Monomeren gebildet, die aus der nachstehenden Liste von Substanzen ausgewählt sind:

- unverzweigte oder verzweigte Alkene mit 15 bis 50, vorzugsweise mit 20 bis 35 Kohlenstoffatomen pro Molekül, bevorzugt $\alpha$-Olefine,
- Ethylen,

- reaktive Polyisobutene, gebildet aus Polyisobutenketten, die am Ende oder in der Nähe des Endes der Polyisobutenkette noch eine reaktionsfähige Doppelbindung aufweisen,
- hydrophobe Vinyl- oder Vinylidenverbindungen, insbesondere Styrol oder
- (Meth)acrylate mit hydrophoben Seitenketten.

Für die Bildung der Struktureinheit(en) A kann somit vorteilhaft von langkettigen α-Olefinen ausgegangen werden. Besonders vorteilhaft ist auch der Einsatz von reaktiven Polyisobutenen, das heißt von Polyisobutenen, die aus Ketten gebildet sind, die am Ende oder in der Nähe des Endes der Kette noch eine reaktionsfähige Doppelbindung aufweisen.

Alle oben aufgeführten Substanzen oder Mischungen von Substanzen sind großtechnische Produkte, und entsprechend preiswert verfügbar.

Zur Bildung der Struktureinheit(en) A', das heißt der Struktureinheit oder der Struktureinheiten, die eine Ankerfunktion zum Anbinden von Seitenketten aufweisen, wird bevorzugt eine Substanz oder eine Mischung von Substanzen, ausgewählt aus der nachstehenden Aufzählung, eingesetzt:

- Maleinsäureanhydrid oder seine Derivate, die vorzugsweise eine polymerisierbare oder alkoxilierbare Seitenkette tragen,
- Vinylalkohole oder ihre Derivate, die vorzugsweise eine polymerisierbare oder alkoxilierbare Seitenkette tragen,
- (Meth)acrolein oder
- (Meth)acrylsäure oder ihre Derivate, die vorzugsweise eine oder mehrere polymerisierbare oder alkoxilierbare Seitenketten tragen.

Unter dem Begriff "Polymerisation" bzw. "polymerisierbar" sollen vorliegend alle Verfahren verstanden werden, mit denen eine polymere Verbindung hergestellt werden kann. Neben den klassischen Verfahren zur Polymerisation sollen insbesondere auch Verfahren wie Polykondensation, Polyaddition einbezogen sein.

Auch hierbei handelt es sich durchwegs um großtechnische, und somit preiswert verfügbare Produkte.

Das die Struktureinheiten X bildende Monomer ist vorteilhaft Ethylenoxid oder eine Mischung aus Ethylenoxid und Propylenoxid, das zur Bildung der Seitenketten zu einem hydrophilen Polyethylenoxid- oder Polyethylenoxid-/Polypropylenoxid-Block weiter umgesetzt wird.

Besonders günstig ist es, die Struktureinheiten X aus einer Mischung von Ethylenoxid und Propylenoxid, bevorzugt mit einem Propylenoxidanteil von 5 bis 20 %, aufzubauen.

Es wurde gefunden, dass für die Effizienzsteigerung von Tensiden besonders wirksame Cotensidstrukturen erhalten werden, wenn man die aus Ethylenoxid oder Ethylenoxid/Propylenoxid-Mischungen gebildeten hydrophilen Seitenketten mit hydrophoben Blöcken endgruppenverschließt, das heißt dass alle oder ein Teil der aus den hydrophilen Ethylenoxid- oder Ethylenoxid-/Propylenoxid-Blöcken gebildeten Seitenketten in jeweils einem hydrophoben Block, vorzugsweise einem hydrophoben Poly- oder Oligoalkylenoxid oder in einer verzweigten oder unverzweigten $C_{10}$- bis $C_{30}$-Alkylkette enden.

Die die Seitenketten bildenden Struktureinheiten X können auch aus einem unverzweigten oder verzweigten Alkyl-, Cycloalkyl-, Aryl- oder Aralkylrest mit 4 bis 400 Kohlenstoffatomen oder einem Polyolefin oder hydrophoben Poly- oder Oligoalkylenoxid-Block gebildet sein.

Der Polyolefinblock kann bevorzugt aus einem oder mehreren der nachfolgenden Monomere: Ethen, Propen, 1-Buten, 2,3-Buten, 2-Methyl-1,2-propen (Isobuten), 1-Penten, 2,3-Penten 2-Methyl-1,2-buten, 3-Methyl-1,2-buten, 2,3-Hexen 3,4-Hexen, 2-Methyl-1,2-penten, 2-Ethyl-1,2-buten, 3-Methyl-1,2-Peten, Decen, 4-Methyl-1,2-penten, Styrol oder aus einer Mischung aus Olefinen technisch verfügbarer Raffinatströme, gebildet sein.

In einer bevorzugten Ausführungsform ist die Molfraktion 1 der die Seitenketten bildenden Struktureinheiten X Null, das die Struktureinheit A bildende Monomer oder mindestens eines der die Struktureinheiten A bildende Monomere trägt eine hydrophobe Seitenkette und die Struktureinheiten A' haben überwiegend hydrophilen Charakter.

Besonders bevorzugt ist eine Mischung, umfassend ein Cotensid, wobei das die Struktureinheit oder Struktureinheiten A' bildende Monomer Maleinsäureanhydrid und das die Struktureinheiten X bildende Monomer Ethylenoxid ist.

Eine weitere bevorzugte Mischung umfasst ein Cotensid, wobei das die Struktureinheit(en) A' bildende Monomer ein alkoxylierbares, vorzugsweise hydroxy-oder aminofunktionalisiertes Maleinsäureimid und die Seitenkette(n) unverzweigte oder verzweigte Alkylreste mit 3 bis 50 Kohlenstoffatomen und/oder Polyethylenoxid- oder Polyethylenoxid-/Polypropylenoxid-Blöcke sind, die vorzugsweise in einem hydrophoben Block, insbesondere einer verzweigten oder unverzweigten $C_{10}$- bis $C_{30}$-Alkylkette, enden.

Bevorzugt ist auch eine Mischung, umfassend ein Cotensid, wobei das die Struktureinheit(en) A' bildende Monomer Vinylalkohol und das die Struktureinheiten X bildende Monomer (Meth)acrylsäure oder Ethylenoxid oder eine Mischung aus Ethylenoxid und Propylenoxid ist.

Weiter bevorzugt ist das die Struktureinheit(en) A' bildende Monomer ein Vinylalkoholderivat mit einer oder mehrerenpolymerisierbaren Seitenketten und das die Struktureinheiten X bildende Monomer Vinylpyrrolidon, (Meth) acrylsäure, Vinylalkohol oder ein Vinylalkoholderivat.

Es ist nicht notwendig, dass alle Struktureinheiten A' mit Ankerfunktion auch jeweils tatsächlich mit einer Seitenkette funktionalisiert sind. Die Erfindung umfasst gleichermaßen auch Kammpolymere, die noch freie Ankergruppen umfassen, wobei die Ankergruppen entweder nicht umgesetzt oder vor der Reaktion geschützt wurden. Es ist somit möglich, durch entsprechende Funktionalisierung lediglich eines Teils der Struktureinheiten A' mit Ankerfunktion ein Polymer mit einer für den speziellen Anwendungsfall geeigneten Dichte an Seitenketten zu synthetisieren.

Für den Fall, dass das die Struktureinheit(en) A' bildende Monomer Maleinsäureanhydrid ist, können die Maleinsäureanhydrid-Einheiten, die keine Seitenkette tragen, als Anhydrid, als Mono- oder Diester, als Amid oder Imid, als freie Säure sowie in teilweise oder vollständig neutralisierter Form vorliegen.

Für den Fall, dass das die Struktureinheit(en) A' bildende Monomer Vinylalkohol ist, können die keine Seitenketten tragenden Vinylalkoholgruppen als freier Alkohol oder als Vinylacetat vorliegen.

Ist das die Struktureinheit(en) A' bildende Monomer (Meth)acrylat, können die nicht funktionalisierten (Meth) acrylateinheiten als Ester, als Amid, als freie Säure sowie in teilweise oder vollständig neutralisierter Form vorliegen.

Die erfindungsgemäße Mischung umfasst neben den vorstehend beschriebenen CoTensiden ein Tensid. Dabei kann es sich auch um eine Mischung von Tensiden handeln. Grundsätzlich kann jedes Tensid, aus jeder der bekannten Tensid-Gruppen, insbesondere ionische oder nichtionische Tenside, oder auch Mischungen von ionischen oder nichtionischen Tensiden eingesetzt werden.

Geeignet sind, je nach Einsatzgebiet der erfindungsgemäßen Mischungen, beispielsweise alle klassischen Reiniger-Tenside, oder lebensmittel-zugelassene Tenside, wie Tweens® oder Spans®. Von den Tensidklassen her sind geeignet nicht-ionische, anionische, kationische, amphotere Tenside; insbesondere auch Polymertenside, Peptidtenside, Silikontenside, aminosäurebasierte Tenside, Zuckertenside, fettbasierte Tenside, Geminitenside, Aminoxide, Amidoaminoxide, Alkylbetaine, Ethercarboxylate, Ampho-Acetate, Alkylsulfate oder Sulfosuccinate.

Der Anteil des Cotensids, bezogen auf das Tensid, liegt bevorzugt im Bereich von 0,01 bis 99,99 %, insbesondere zwischen 1 und 50 %, besonders bevorzugt zwischen 5 und 25 %.

Geeignete anionische Tenside sind beispielsweise Fettalkoholsulfate von Fettalkoholen mit 8 bis 22, vorzugsweise 10 bis 18 Kohlenstoffatomen, zum Beispiel $C_9$- bis $C_{11}$-Alkoholsulfate, $C_{12}$- bis $C_{13}$-Alkoholsulfate, Cetylsulfat, Myristylsulfat, Palmitylsulfat, Stearylsulfat und Talgfettalkoholsulfat.

Weitere geeignete anionische Tenside sind sulfatierte ethoxylierte $C_8$- bis $C_{22}$-Alkohole (Alkylethersulfate) bzw. deren lösliche Salze. Verbindungen dieser Art werden beispielsweise dadurch hergestellt, das man zunächst einen $C_8$- bis $C_{22}$-, vorzugsweise einen $C_{10}$-bis $C_{18}$-Alkohol, zum Beispiel einen Fettalkohol, alkoxyliert und das Alkoxylierungsprodukt anschließend sulfatiert. Für die Alkoxylierung verwendet man vorzugsweise Ethylenoxid, wobei man pro Mol Fettalkohol 2 bis 50, vorzugsweise 3 bis 20 mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte $C_8$- bis $C_{22}$-Alkohole, die Ethylenoxid und Propylenoxid oder Ethylenoxid und Butylenoxid enthalten. Die alkoxylierten $C_8$- oder bis $C_{22}$-Alkohole können die Ethylenoxid-, Propylenoxid- und butylenoxideinheiten in Form von Blöcken oder in statistischer Verteilung enthalten.

Geeignet sind auch Alkansulfonate, wie $C_8$- bis $C_{24}$-, vorzugsweise $C_{10}$- bis $C_{18}$-Alkansulfonate sowie Seifen, wie Na oder K-Salze von $C_8$- bis $C_{24}$-Carbonsäuren.

Weitere geeignete anionische Tenside sind N-Acylsarkosinate mit aliphatischen gesättigten oder ungesättigten $C_8$- bis $C_{25}$-Acylresten, vorzugsweise $C_{10}$- bis $C_{20}$-Acylresten, zum Beispiel N-Oleoylsarkosinat.

Weiterhin können die erfindungsgemäßen Mischungen $C_{10}$- bis $C_{13}$-lineare und/oder -leicht verzweigte Alkylbenzolsulfonate (LAS) enthalten.

Die anionischen Tenside werden der Mischung, vorzugsweise in Form von Salzen zugegeben. Geeignete Kationen in diesen Salzen sind Alkalimetallsalze wie Natrium, Kalium und Lithium und Ammoniumsalze wie zum Beispiel Hydroxyethylammonium-, Di(hydroxyethyl)ammonium- und Tri(hydroxyethyl)ammoniumsalze.

Geeignete nichtionische Tenside sind insbesondere:

- alkoxylierte $C_8$- bis $C_{22}$-Alkohole wie Fettalkoholalkoxylate oder Oxoalkoholalkoxylate. Diese können mit Ethylenoxid, Propylenoxid und/oder Butylenoxid alkoxyliert sein. Als Tenside einsetzbar sind hierbei sämtliche alkoxylierten Alkohole, die mindestens zwei Moleküle eines der vorstehend genannten Alkylenoxide addiert enthalten. Hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Die nichtionischen Tenside enthalten pro Mol Alkohol im allgemeinen 2 bis 50, vorzugsweise 3 bis 20 Mol mindestens eines Alkylenoxids. Vorzugsweise enthalten diese als Alkylenoxid Ethylenoxid. Die Alkohole haben vorzugsweise 10 bis 18 Kohlenstoffatome. Je nach Art des bei der Herstellung verwendeten Alkoxylierungskatalysators weisen die Alkoxylate eine breite oder enge Alkylenoxid-Homologenverteilung auf;
- Alkylphenolalkoxylate wie Alkylphenolethoxylate mit $C_6$- bis $C_{14}$-Alkylketten und 5 bis 30 Alkylenoxideinheiten;

- Alkylpolyglucoside mit 8 bis 22, vorzugsweise 10 bis 18 Kohlenstoffatomen in der Alkylkette und im allgemeinen 1 bis 20, vorzugsweise 1,1 bis 5 Glucosideinheiten Sorbitanalkanoate, auch alkoxyliert;
- N-Alkylglucamide, Fettsäurealkoxylate, Fettaminalkoxylate, Fettsäureamidalkoxylate, Fettsäurealkanolamidalkoxylate, alkoxyliert, Blockcopolymere aus Ethylenoxid, Propylenoxid und/oder Butylenoxid, Polyisobuten-Ethoxylate sowie Polyisobuten-Maleinsäureanhydrid-Derivate, Monoglyceride sowie Bisglyceride, auch alkoxyliert.

Besonders geeignete nichtionische Tenside sind Alkylalkoxylate oder Gemische von Alkylalkoxylaten, wie sie beispielsweise in DE-A 102 43 363, DE-A 102 43 361, DE-A 102 43 360, DE-A 102 43 365, DE-A 102 43 366, DE-A 102 43 362 oder in DE-A 43 25 237 beschrieben sind. Hierbei handelt es sich um Alkoxylierungsprodukte, die durch Umsetzung von Alkanolen mit Alkylenoxiden in Gegenwart von Alkoxylierungskatalysatoren erhalten wurden oder um Gemische von Alkoxylierungsprodukten. Besonders geeignete Starteralkohole sind die sogenannten Guerbet-Alkohole, insbesondere Ehtylhexanol, Propylheptanol und Butyloktanol. Besonders bevorzugt ist Propylheptanol. Bevorzugte Alkylenoxide sind Propylenoxid und Ethylenoxid, wobei Alkylalkoxylate mit direkter Anbindung eines bevorzugt kurzen Poylypropylenoxidblocks an den Starteralkohol, wie sie beispielsweise in DE-A 102 43 365 beschrieben sind, insbesondere aufgrund ihres geringen Restalkoholgehalts und ihrer guten biologischen Abbaubarkeit bevorzugt sind.

Als Alkoxylierungskatalysatoren können Basen eingesetzt werden, beispielsweise Alkalihydroxide oder Alkalialkoholate jedoch auch Lewis-Säuren, beispielsweise $BF_3$, $SbCl_5$, $SnCl_4$ x $2H_2O$, $BF_3$ x $H_3BO_4$, oder $BF_3$-Dietherat. Besonders geeignete Alkoxylierungskatalysatoren sind Doppelhydroxid-Tone wie Hydrotalkit, die insbesondere mit Additiven modifiziert sein können, wie in DE-A 43 25 237 beschrieben.

Je nach Wahl des Alkoxylierungskatalysators resultieren jeweils spezifische Eigenschaften der Alkoxylate, insbesondere bezüglich der Verteilung des Alkoxylierungsgrades. So werden bei Verwendung der letztgenannten Doppelhydroxid-Tone Alkoxylierungsprodukte mit einer engen Molekulargewichtsverteilung bzw. Homologenverteilung erhalten, die für den Einsatz in den erfindungsgemäßen Mischungen mit Cotensiden besonders geeignet sind.

Die vorstehend beschriebenen vorteilhaften Eigenschaften, insbesondere bezüglich des Alkoxylierungsgrades, werden auch durch Einsatz von Doppelmetallcyanid (DMC)-Verbindungen erreicht, wie sie beispielsweise in DE-A 102 43 361 als Alkoxylierungskatalysatoren beschrieben sind.

Gegenstand der Erfindung ist auch die Verwendung einer Mischung, enthaltend ein Tensid und ein vorstehend beschriebenes Cotensid mit der Struktur eines amphiphilen Kammpolymers zum Stabilisieren von Emulsionen, insbesondere von Öl/Wasser-Emulsionen, Wasser/Öl-Emulsionen, Mikroemulsionen oder multiplen Emulsionen wie Öl/Wasser/Öl-Emulsionen oder Wasser/Öl/Wasser-Emulsionen. Stabilisieren bedeutet im vorliegenden Zusammenhang, dass durch Zusatz von Cotensiden die Effizienz von Tensiden gesteigert wird, das heißt die Solubilisierung eines definierten Öl/Wasser-Gemisches unter definierten Bedingungen mit einer geringeren Menge an Tensid ermöglicht wird.

Besonders bevorzugt eignen sich die vorstehend beschriebenen Cotenside mit der Struktur amphiphiler Kammpolymere zur Stabilisierung von Mikroemulsionen, das heißt zur Verschiebung des sogenannten X-Punktes, der die niedrigste Konzentration an Tensid bei gegebener Temperatur darstellt, ab der der thermodynamische Zustand der Mikroemulsion, das heißt der in makroskopischer Betrachtung einphasige Zustand, eintritt, zu niedrigeren Tensidkonzentrationen.

Die erfindungsgemäßen Mischungen sind grundsätzlich in allen Bereichen einsetzbar, in denen Emulsionen eine Rolle spielen, beispielsweise in den in DE-A 101 18 480 aufgeführten Anwendungsbereichen für Mischungen umfassend ein Tensid und ein AB-Blockcopolymerisat als Additiv (Cotensid), die daneben Zusatzstoffe enthalten, deren Effizienz durch das Tensid/Additivsystem gesteigert werden kann: Beispielsweise als Pflanzenstärkungs-, -wuchs- oder Pflanzenschutzmittel, Produkte mit mikrobioziden Wirkstoffen, Produkte mit positiv oder negativ wirkenden Mikroorganismen, insbesondere mit einem Gehalt an Enzymen, Reinigungs- und/oder Pflegemittel für den Haushalt und für gewerbliche Zwecke, Desinfektionsmittel, Haar-, Körperpflege- oder Reinigungsmittel, Fahrzeugreinigungs-, -pflege- und/oder -konservierungsmittel, Textilbehandlungsmittel, Leder- und/oder Pelzpflegemittel, als Farben, Lacke, Arzneimittel, Bauhilfsstoffe, Zahnpasten oder Mundspülmittel.

Synergistische Effekte, wie sie in DE-A 101 18 480 für das System Tensid/AB-Blockcopolymerisat in Verbindung mit zusätzlichen Bioziden, Mikroorganismen und/oder beliebigen anderen Wirkstoffen beschrieben sind, werden entsprechend für Systeme enthaltend die erfindungsgemäßen Mischungen umfassend ein Tensid und ein Cotensid sowie entsprechende Zusatzstoffe, insbesondere Biozide, Mikroorganismen und/oder beliebige andere Wirkstoffe, erreicht.

Besonders geeignet ist der Einsatz der erfindungsgemäßen Mischungen in Zubereitungen für die Verwendung in der Kosmetik, Pharmazie sowie im Nahrungsmittelbereich, enthaltend mindestens ein Retinoid, mindestens ein wasserlösliches Antioxidans und mindestens ein öllösliches Oxidans, wie sie in der nicht vorveröffentlichten deutschen Patentanmeldung DE 102 337 40.3, beschrieben sind, deren Offenbarungsgehalt hiermit voll umfänglich in die vorliegende Patentanmeldung einbezogen wird. Hierbei handelt es sich um kosmetische sowie dermatologische oder pharmazeutische Zubereitungen, die in der Regel auf der Basis eines Trägers aufgebaut sind, der mindestens eine Ölphase enthält. Demgemäß kommen Öle, Cremes, Pasten oder fettfreie Gele oder bevorzugt Emulsionen in Betracht.

Die genannten Zubereitungen enthalten pro Gewichtsteil Retinoid mindestens ein Gewichtsteil eines oder mehrerer wasserlöslicher Antioxidantien und 0,1 bis 100 Gewichtsteile eines oder mehrerer öllöslicher Antioxidantien, wobei der Gehalt an einem oder mehreren wasserlöslichen Antioxidantien im Bereich von 0,05 bis 0,8 Gew.-% liegt, bezogen auf die Gesamtmenge der Zubereitungen.

Unter Retinoiden sind Vitamin A Alkohol (Retinol) und seine Derivate wie Vitamin A Aldehyd (Retinal), Vitamin A Säure (Retinsäure) und Vitamin A Ester wie Retinylacetat und Retinylpalmitat gemeint. Der Begriff Retinsäure umfasst dabei sowohl all-trans Retinsäure als auch 13-cis Retinsäure. Die Begriffe Retinol und Retinal umfassen bevorzugt die all-trans Verbindungen. Als bevorzugtes Retinoid verwendet man für die erfindungsgemäßen Zubereitungen all-trans-Retinol.

Als wasserlösliche Antioxidantien sind u.a. Ascorbinsäure, Natriumsulfit, Natriummetabisulfit, Natriumbisulfit, Natriumthiosulfit, Natriumformaldehydsulfoxylat, Isoascorbinsäure, Thioglycerin, Thiosorbit, Thioharnstoff, Thioglykolsäure, Cysteinhydrochlorid, 1,4-Diazobicyclo-(2,2,2)-oktan oder Mischungen davon gemeint.

Bevorzugte wasserlösliche Antioxidantien sind Ascorbinsäure (L-Ascorbinsäure) und Isoascorbinsäure (D-Ascorbinsäure), besonders bevorzugt L-Ascorbinsäure.

Bei der besonders bevorzugt verwendeten L-Ascorbinsäure kann es sich um die freie Säure aber auch um deren Salze handeln. Beispiele für Salze der L-Ascorbinsäure sind Alkali- oder Erdalkalimetallsalze der L-Ascorbinsäure wie Natrium-L-ascorbat, Kalium-L-ascorbat oder Calcium-L-ascorbat, aber auch Salze der L-Ascorbinsäure mit einer organischen Aminverbindung wie Cholinascorbat oder L-Carnitinascorbat. Ganz besonders bevorzugt verwendet man die freie L-Ascorbinsäure oder Natrium-L-ascorbat. Entsprechendes gilt für die Verwendung von D-Ascorbinsäure.

Als öllösliche Antioxidantien sind u.a. butyliertes Hydroxytoluol (BHT), Ascorbylpalmitat, butyliertes Hydroxyanisol, $\alpha$-Tocopherol, Phenyl-$\alpha$-naphthylamin oder Mischungen davon gemeint.

Bevorzugt öllösliches Antioxidans ist $\alpha$-Tocopherol, wobei es sich dabei sowohl um (R,R,R)- als auch um (all-rac)-$\alpha$-Tocopherol handeln kann.

Zu den kosmetischen oder pharmazeutischen Zubereitungen kommen übliche Hilfsstoffe in Betracht, beispielsweise Co-Emulgatoren, Fette und Wachse, Staltisatoren, Verdickungsmittel, biogene Wirkstoffe, Filmbildner, Duftstoffe, Farbstoffe, Perlglanzmittel, Konservierungsmittel, Pigmente, Elektrolyte (zum Beispiel Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind unter anderem Bienenwachs, Paraffinwachs oder Mikrowachs gegebenenfalls in Kombination mit hydrophilen Wachsen zu nennen. Als Stabilisatoren können Metallsalze von Fettsäuren wie zum Beispiel Magnesium-, Aluminium- und/oder Zinksteaat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre CelluloseDerivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkomission der Deutschen Forschungsgemeinschaft, veröffentlicht im Verlag Chemie, Weinheim 1984, zusammengestellt sind. Diese Farbstoffe werden üblicherweise in Konzentrationen von 0,001 bis 0,1 Gew.-%, bezogen auf die gesamte Mischung, eingesetzt.

Die erfindungsgemäßen Mischungen können auch vorteilhaft in kosmetischen oder dermatologischen Zubereitungen eingesetzt werden, enthaltend mindestens einen UV-Filter, besonders bevorzugt in kosmetischen oder dermatologischen Zubereitungen enthaltend als UV-B-Filter 2,4,6-Trianilino-p-(carbo-2'-ethyl-hexyl-1'-oxi)-1,3,5-triazin, das von der BASF AG unter der Warenbezeichnung Uvinul® T150 vermarktet wird und als UV-A-Filter N,N-Diethylamino-hydroxybenzoyl-n-hexylbenzoat, das von der BASF AG unter der Warenbezeichnung Uvinul® A Plus vermarktet wird.

Besonders vorteilhaft können die erfindungsgemäßen Mischungen in kosmetischen oder dermatologischen Zubereitungen enthalten sein, wie sie in der nicht vorveröffentlichten deutschen Patentanmeldung DE 102 00 400 7885.8 beschrieben sind, die hiermit voll umfänglich in den Offenbarungsgehalt der vorliegenden Patentanmeldung einbezogen wird.

In der genannten Patentanmeldung wird eine Kombination aus Uvinul® T150, Uvinul® A Plus zusammen mit Zinkoxid und/oder Titandioxid beschrieben, wobei zusätzlich UV-A- und UV-B-Filtersubstanzen aus der nachfolgenden Tabelle enthalten sein können:

| Nr. | Stoff | CAS-Nr. (= Säure) |
|---|---|---|
| 1 | 4-Aminobenzoesäure | 150-13-0 |
| 2 | 3-(4'Trimethylammonium-benzylidenboman-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium-u. Triethanolaminsalze | 27503-81-7 |
| 6 | 3,3'-(1,4-Phenylendimethin)-bis (7,7-dimethyl-2-oxobicyclo[2.2.1]heptan-1-methansulfon- | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'-Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino] carbonyl]phenyl]amino]-1,3,5-triazin-2,4-diyl]diimino]bis-,bis(2-ethylhexyl)ester | 154702-15-5 |
| 30 | 2-(2H-Benzotriazol-2-yl)-4-methyl-6-[2-methyl-3-[1,3,3,3-tetramethyl-1-1[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexal)oxyl]] (Aniso Triazin) | 187393-00-6 |

Die Lichtschutzmittel enthaltenden kosmetischen und dermatologischen Zubereitungen sind in der Regel auf der Basis eines Trägers, der mindestens eine Ölphase enthält. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wässrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure sowie Siliconöle.

Geeignete Siliconöle sind zum Beilspiel lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugt cyclische Siloxane weisen 4- bis 8-gliedrige Ringe auf. Geeignete cyclische Siloxane sind zum Beispiel unter der Bezeichung Cyclomethicon kommerziell erhältlich.

Weiter ist auch eine Mikroemulsion, enthaltend ein Tensid und ein Cotensid Gegenstand der Erfindung, wobei man als Cotensid eine Substanz oder eine Mischung von Substanzen mit der Struktur eines amphiphilen Kammpolymers, wie vorstehend beschrieben, einsetzt. Hierbei kann es sich um bikontinuierliche Mikroemulsionen handeln, nichtbikontinuierliche Mikroemulsionen sind jedoch gleichermaßen von der Erfindung umfasst.

Die erfindungsgemäßen Mischungen sind optimal geeignet für die Aufnahme und Abgabe von Hydrophoben, insbesondere die Verwendung als Waschmittel, Emulgator, Schaumregulierer, Netzmittel für harte Oberflächen sowie als Reaktionsmedium für organische, anorganische, bioorganische oder photochemische Reaktionen.

Bevorzugt ist die Verwendung in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

Gegenstand der Erfindung sind auch Wasch-, Reinigungs-, Desinfektions-, Netz-, Beschichtungs-, Klebe-, Lederentfettungs-, Feuchthalte- oder Textilbehandlungsmittel oder pharmazeutische, Lebensmittel-, Pflanzenschutz- oder kosmetische Formulierung, insbesondere Sonnenschutz-, Hautpflege- oder Haarstylingmittel, Duschgele, Shampoos, Badezusätze oder Duftöle, enthaltend neben üblichen Inhaltsstoffen eine Mischung umfassend ein Tensid und ein wie vorstehend beschriebenes Cotensid oder eine Mikroemulsion umfassend ein Tensid und ein Cotensid.

Die Erfindung wird im Folgenden anhand von Beispielen näher erläutert.

**Herstellungsbeispiele 1 bis 3**

In einem Polymerisationsreaktor aus Stahl mit Rührer und Dosiereinrichtungen, wurden 1195 g eines $C_{20}$-$C_{24}$-$\alpha$-Olefin-Gemisches vorgelegt und unter Rühren in einem schwachen Stickstoffstrom auf 190°C erhitzt. Sobald diese Temperatur erreicht war, fügte man gleichmäßig 392 g auf 70°C erhitztes Maleinsäureanhydrid und davon getrennt 16 g Di-tert.-butylperoxid innerhalb von 4 Stunden zu. Es handelt sich somit um eine radikalische Polymerisation zur Herstellung des Rückgrats eines beispielhaften Kammpolymers. Anschließend wurde das Reaktionsgemisch zwei Stunden lang bei 190°C gerührt und danach zur Anbindung der Seitenketten verestert.

Hierzu wurde unter Rühren bei 150°C ein Alkylpolyglykolether aus einem $C_{18}$-Oxoalkohol mit Ethylenoxid-Blöcken der folgenden Länge und in der jeweils angegebenen Menge:

Beispiel 1   11 EO-Einheiten, 1450 g,
Beispiel 2   25 EO-Einheiten, 2665 g und
Beispiel 3   80 EO-Einheiten, 6878 g,

zugeführt und fünf Stunden lang gerührt. Danach wurde das Reaktionsgemisch unter Rühren auf 90°C abgekühlt. Innerhalb einer halben Stunde gab man dann jeweils getrennt voneinander 160 g einer 50 %igen wässrigen Natronlauge und soviel auf 90°C erwärmtes Wasser zu, dass sich eine Lösung mit einem Feststoffgehalt von 30 % ergab. Das Reaktionsgemisch wurde 4 Stunden lang im Temperaturbereich von 90 bis 95°C gerührt und danach auf Umgebungstemperatur abgekühlt. Man erhielt auf diese Weise eine schwach viskose wässrige Dispersion eines Copolymers, bei dem 50 Mol-% der insgesamt entstehenden Carboxylgruppen neutralisiert waren.

**Anwendungsbeispiele**

In der nachfolgenden Figur ist die Verschiebung des X-Punktes, das heißt der Mindestkonzentration an Tensid bei gegebener Temperatur dargestellt, ab der für das Referenzsystem Wasser/n-Dekan und ein gegebenes Tensid (Lutensol® ON 50 der BASF AG) die Wasser- und die n-Dekan-Phase vollständig mischbar sind und eine stabile Mikroemulsion entsteht, bei Zugabe der folgenden Cotenside:

**Anwendungsbeispiel 1**

Copolymer aus Maleinsäureanhydrid und $C_{20}$-$C_{24}$-$\alpha$-Olefin, bei dem die Hälfte der Maleinsäureanhydrid-Untereinheiten mit Pluriol® A 750 I der BASF AG, das heißt einem Methylpolyethylenglykol mit der mittleren Molekularmasse von 750 g/Mol verestert wurden.

**Anwendungsbeispiel 2**

Copolymer aus Maleinsäureanhydrid und $C_{20}$-$C_{24}$-$\alpha$-Olefin, bei dem die Hälfte der Maleinsäureanhydrid-Untereinheiten mit Lutensol® AT 11 der BASF AG, das heißt ein $C_{16}$-$C_{18}$-Fettalkohol Ethoxylat mit 11 Ethylenoxideinheiten verestert wurden.

Jedes der Cotenside wurde jeweils in einem Anteil von 10 Gew.-% dem Tensid Lutensol® ON 50 der BASF AG, das heißt einem $C_{10}$-Oxoalkoholethoxylat mit 5 EthylenoxidEinheiten, zugegeben.

Der X-Punkt lag beim Vergleichsbeispiel ohne Cotensid bei 22,5 % Lutensol® ON 50 bei 64°C, im Anwendungsbeispiel 1, bei Zusatz von Cotensid, bei 20 % Lutensol® ON 50, bei 65°C und im Anwendungsbeispiel 2, unter Zusatz von Cotensid, bei 15 % Lutensol® ON 50, bei 70°C.

In der anliegenden Figur ist auf der Abszisse die Konzentration des Tensids Lutensol® ON 50, in der Fig. mit $c_{Tensid}$, in Gew.-% und auf der Ordinate die Temperatur in °C dargestellt. Ausschnitte aus den jeweiligen Phasendiagrammen sind für das System Wasser/n-Dekan 1 : 1 und dem oben genannten Tensid unter I zum Vergleich, das heißt ohne Zusatz eines Cotensids, unter II für das erfindungsgemäße Anwendungsbeispiel 1 und unter III für das erfindungsgemäße Anwendungsbeispiel 2 angegeben. Die Graphik verdeutlicht die Verschiebung des X-Punktes in den Anwendungsbeispielen nach der Erfindung (Darstellung in II bzw. III) gegenüber dem Stand der Technik (Darstellung I).

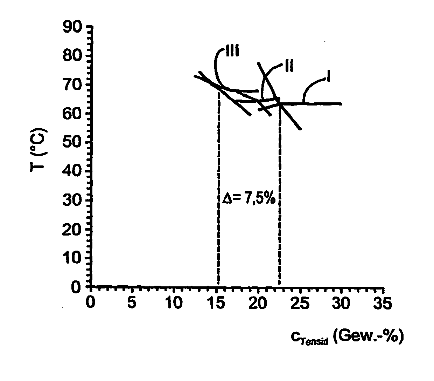

**Patentansprüche**

1.    Mischung, enthaltend ein Tensid und ein Cotensid, wobei man als Cotensid ein amphiphiles Kammpolymer einsetzt, aufweisend ein Rückgrat mit am Rückgrat angebrachten zwei oder mehreren Seitenketten, wobei sich die Seitenketten untereinander und/oder die Seitenketten vom Rückgrat in ihrem amphiphilen Charakter unterscheiden, **dadurch gekennzeichnet, dass** das Kammpolymer sich wiederholende, jeweils eine oder mehrere Struktureinheiten A, A' und X enthält, wobei die Struktureinheiten A und A' das Rückgrat bilden und die Struktureinheiten A' eine Ankerfunktion zum Anbinden der die Seitenketten bildenden Struktureinheiten X haben, und wobei die Struktureinheiten A in einer Molfraktion n, die Struktureinheiten A' in einer Molfraktion m und die Struktureinheiten X in einer Molfraktion 1 vorliegen, mit

$$n + m + l = 1,$$

$$n \geq m$$

und

$$l > m \text{ oder } l = 0,$$

wobei
das die Struktureinheiten A bildende Monomer (die die Struktureinheiten A bildenden Monomere) eine Substanz oder eine Mischung von Substanzen, ausgewählt aus der nachstehenden Aufzählung, ist (sind):

- unverzweigte oder verzweigte Alkene mit 3 bis 50, vorzugsweise mit 10 bis 35 Kohlenstoffatomen pro Molekül, bevorzugt $\alpha$-Olefine,

- Ethylen,
- reaktive Polyisobutene, gebildet aus Polyisobutenketten, die am Ende oder in der Nähe des Endes der Polyisobutenkette noch eine reaktionsfähige Doppelbindung aufweisen,
- hydrophobe Vinyl- oder Vinylidenverbindungen, insbesondere Styrol oder
- (Meth)acrylate mit hydrophoben Seitenketten, und

das die Struktureinheiten A' bildende Monomer (die die Struktureinheiten A' bildenden Monomere) eine Substanz oder eine Mischung von Substanzen, ausgewählt aus der nachstehenden Aufzählung, ist (sind):

- Maleinsäureanhydrid oder seine Derivate, sofern sie polymerisierbare oder alkoxylierbare Seitenketten tragen,
- Vinylalkohole oder ihre Derivate, die vorzugsweise eine polymerisierbare oder alkoxilierbare Seitenkette tragen,
- (Meth)acrolein oder
- (Meth)acrylsäure oder ihre Derivate, die vorzugsweise eine oder mehrere polymerisierbare oder alkoxilierbare Seitenketten tragen.

2. Mischung nach Anspruch 1, **dadurch gekennzeichnet, dass** das Cotensid eine mittlere Molekularmasse im Bereich von 500 bis 100.000 g/mol, bevorzugt im Bereich von 1000 bis 50.000 g/mol, aufweist.

3. Mischung nach Anspruch 1 oder 2, **dadurch gekennzeichnet, dass** das die Struktureinheiten A bildende Monomer (die die Struktureinheiten A bildenden Monomere) eine oder mehrere hydrophobe Seitenketten trägt (tragen).

4. Mischung nach einem der Ansprüche 1 bis 3, **dadurch gekennzeichnet, dass** die aus den Struktureinheiten X gebildeten Seitenketten hydrophile Polyethylenoxid- oder Polyethylenoxid-/Polypropylenoxid-Blöcke sind.

5. Mischung nach Anspruch 4, **dadurch gekennzeichnet, dass** das die Struktureinheiten X bildende Monomer (die die Struktureinheit X bildenden Monomere) eine Mischung aus Ethylenoxid und Propylenoxid, bevorzugt mit einem Propylenoxidanteil von 5 bis 20 %, ist (sind).

6. Mischung nach Anspruch 4 oder 5, **dadurch gekennzeichnet, dass** alle oder ein Teil der aus den hydrophilen Ethylenoxid- oder Ethylenoxid-/Propylenoxid-Blöcken gebildeten Seitenketten in jeweils einem hydrophoben Block, vorzugsweise einem hydrophoben Poly- oder Oligoalkylenoxid oder in einer verzweigten oder unverzweigten $C_{10}$- bis $C_{30}$-Alkylkette enden.

7. Mischung nach einem der Ansprüche 1 bis 10, **dadurch gekennzeichnet, dass** das die Struktureinheit(en) A' bildende Monomer Maleinsäureanhydrid und das die Struktureinheiten X bildende Monomer Ethylenoxid ist.

8. Mischung nach einem der Ansprüche 1 bis 6, **dadurch gekennzeichnet, dass** das die Struktureinheit(en) A' bildende Monomer Vinylalkohol ist und das die Struktureinheiten X bildende Monomer (Meth)acrylsäure oder Ethylenoxid oder eine Mischung aus Ethylenoxid und Propylenoxid ist.

9. Verwendung einer Mischung nach einem der Ansprüche 1 bis 10 zum Stabilisieren von Emulsionen, insbesondere von Öl/Wasser-Emulsionen, Wasser/Öl-Emulsionen, Mikroemulsionen oder multiple Emulsionen wie Öl/Wasser/ Öl-Emulsionen oder Wasser/Öl/Wasser-Emulsionen.

10. Mikroemulsion, enthaltend ein Tensid und ein Cotensid, **dadurch gekennzeichnet, dass** man als Cotensid eine Substanz oder eine Mischung von Substanzen einsprechend der Definition in einem der Ansprüche 1 bis 8 einsetzt.

11. Verwendung einer Mischung nach einem der Ansprüche 1 bis 8 oder einer Mikroemulsion nach Anspruch 17 als Waschmittel, Emulgator, Schaumregulierer, Netzmittel für harte Oberflächen oder als Reaktionsmedium für organische, anorganische, bioorganische oder photochemische Reaktionen.

12. Verwendung nach Anspruch 11 in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

13. Wasch-, Reinigungs-, Desinfektions-, Netz-, Beschichtungs-, Klebe-, Lederentfettungs-, Feuchthalte- oder Textilbehandlungsmittel oder pharmazeutische, Lebensmittel-, Pflanzenschutz- oder kosmetische Formulierung, insbesondere Sonnenschutz-, Hautpflege- oder Haarstylingmittel, Duschgele, Shampoos, Badezusätze oder Duftöle, enthaltend neben üblichen Inhaltsstoffen eine Mischung nach einem der Ansprüche 1 bis 8 oder eine Mikroemulsion nach Anspruch 10.

**Claims**

1. A mixture comprising a surfactant and a cosurfactant, where the cosurfactant used is an amphiphilic comb polymer having a backbone with two or more side chains attached to the backbone, where the side chains differ from one another and/or the side chains differ from the backbone in their amphiphilic character wherein the comb polymer comprises repeat, in each case one or more, structural units A, A' and X, where the structural units A and A' form the backbone and the structural units A' have an anchor function for binding the side-chain-forming structural units X and where the structural units A are present in a mole fraction n, the structural units A' are present in a mole fraction m and the structural units X are present in a mole fraction 1, where

$$n + m + 1 = 1,$$

$$n \geq m$$

and

$$1 > m \text{ or } 1 = 0,$$

where
the monomer(s) forming the structural units A is (are) a substance or a mixture of substances chosen from the list below:

- unbranched or branched alkenes having 3 to 50, preferably having 10 to 35, carbon atoms per molecule, preferably $\alpha$-olefins,
- ethylene,
- reactive polyisobutenes, formed from polyisobutene chains, which also have a reactive double bond at the end or in the vicinity of the end of the polyisobutene chain,
- hydrophobic vinyl or vinylidene compounds, in particular styrene, or
- (meth)acrylates with hydrophobic side chains, and

the monomer(s) forming the structural units A' is (are) a substance or a mixture of substances chosen from the list below:

- maleic anhydride or its derivatives, provided they carry polymerizable or alkoxylatable side chains,
- vinyl alcohols or their derivatives, which preferably carry a polymerizable or alkoxylatable side chain,
- (meth)acrolein or
- (meth)acrylic acid or its derivatives, which preferably have one or more polymerizable or alkoxylatable side chains.

2. The mixture according to claim 1, wherein the cosurfactant has an average molecular mass in the range from 500 to 100 000 g/mol, preferably in the range from 1000 to 50 000 g/mol.

3. The mixture as claimed in claim 1 or 2, wherein the monomer(s) forming the structural units A carries (carry) one or more hydrophobic side chains.

4. The mixture as claimed in any of claims 1 to 3, wherein the side chains formed from the structural units X are hydrophilic polyethylene oxide or polyethylene oxide/polypropylene oxide blocks.

5. The mixture as claimed in claim 4, wherein the monomer forming the structural units X (the monomers forming the structural unit X) is (are) a mixture of ethylene oxide and propylene oxide, preferably with a propylene oxide content of from 5 to 20%.

6. The mixture as claimed in claim 4 or 5, wherein a11 or some of the side chains formed from the hydrophilic ethylene oxide or ethylene oxide/propylene oxide blocks end in each case in a hydrophobic block, preferably a hydrophobic poly- or oligoalkylene oxide, or in a branched or unbranched $C_{10}$- to $C_{30}$-alkyl chain.

7. The mixture as claimed in any of claims 1 to 6, wherein the monomer forming the structural unit(s) A' is maleic anhydride and the monomer forming the structural units X is ethylene oxide.

8. The mixture as claimed in any of claims 1 to 6, wherein the monomer forming the structural unit(s) A' is vinyl alcohol and the monomer forming the structural units X is (meth)acrylic acid or ethylene oxide or a mixture of ethylene oxide and propylene oxide.

9. The use of a mixture as claimed in any of claims 1 to 8 for stabilizing emulsions, in particular oil/water emulsions, water/oil emulsions, microemulsions or multiple emulsions such as oil/water/oil emulsions or water/oil/water emulsions.

10. A microemulsion comprising a surfactant and a cosurfactant, wherein the cosurfactant used is a substance or a mixture of substances corresponding to the definition in any of claims 1 to 8.

11. The use of a mixture as claimed in any of claims 1 to 8 or of a microemulsion as claimed in claim 10 as detergent, emulsifier, foam regulator, wetting agent for hard surfaces or as reaction medium for organic, inorganic, bioorganic or photochemical reactions.

12. The use as claimed in claim 11 in detergents, surfactant formulations for the cleaning of hard surfaces, humectants, cosmetic, pharmaceutical and crop protection formulations, paints, coatings, adhesives, leather degreasing compositions, formulations for the textile industry, fiber processing, metal processing, food industry, water treatment, paper industry, fermentation, mineral processing, fire protection or in emulsion polymerizations.

13. A detergent, cleaner, disinfectant, wetting agent, coating, adhesive, leather degreasing composition, humectant or textile-treatment composition or a pharmaceutical, food, crop protection or cosmetic formulation, in particular sunscreen, skincare or hairstyling composition, shower gel, shampoo, bath additive or scent oil, comprising, as well as customary ingredients, a mixture as claimed in any of claims 1 to 8 or a microemulsion as claimed in claim 10.

**Revendications**

1. Mélange, contenant un tensioactif et un co-tensioactif, dans lequel on utilise comme co-tensioactif un polymère en peigne amphiphile comportant un squelette avec deux ou plus de deux chaînes latérales placées sur le squelette, les chaînes latérales différant l'une de l'autre et/ou les chaînes latérales différant du squelette quant à leur caractère amphiphile, **caractérisé en ce que** le polymère en peigne contient un ou plusieurs de chacun des motifs structuraux répétitifs A, A' et X, les motifs structuraux A et A' constituant le squelette et les motifs structuraux A' ayant une fonction d'ancrage pour la fixation des motifs structuraux X constituant les chaînes latérales, et les motifs structuraux A étant présents en une fraction molaire n, les motifs structuraux A' étant présents en une fraction molaire m et les motifs structuraux X étant présents en une fraction molaire l, où

$$n + m + l = 1,$$

$$n \geq m$$

et

$$1 > m \ ou \ l = 0,$$

le monomère constituant les motifs structuraux A (les monomères constituant les motifs structuraux A) étant une substance ou un mélange de substances choisie(s) dans la liste suivante :

- des alcènes ramifiés ou non ramifiés ayant de 3 à 50, de préférence de 10 à 35 atomes de carbone par molécule, de préférence des α-oléfines,
- l'éthylène,
- des polyisobutènes réactifs, constitués de chaînes de polyisobutène, qui comportent encore une double liaison réactive à l'extrémité ou au voisinage de l'extrémité de la chaîne de polyisobutène,
- des composés vinyliques ou vinylidène hydrophobes, en particulier le styrène ou des (méth)acrylates à chaînes latérales hydrophobes, et

le monomère constituant les motifs structuraux A' (les monomères constituant les motifs structuraux A') étant une substance ou un mélange de substances choisie(s) dans la liste suivante :

- l'anhydride maléique ou ses dérivés, dans la mesure où ils portent des chaînes latérales polymérisables ou susceptibles d'alcoxylation,
- des alcools vinyliques ou leurs dérivés, qui portent de préférence une chaîne latérale polymérisable ou susceptible d'alcoxylation,
- la (méth)acroléine ou
- l'acide (méth)acrylique ou ses dérivés, qui portent de préférence une ou plusieurs chaînes latérales polymérisables ou susceptibles d'alcoxylation.

2. Mélange selon la revendication 1, **caractérisé en ce que** le co-tensioactif présente une masse moléculaire dans la plage de 500 à 100 000 g/mole, de préférence dans la plage de 1 000 à 50 000 g/mole.

3. Mélange selon la revendication 1 ou 2, **caractérisé en ce que** le monomère constituant les motifs structuraux A (les monomères constituant les motifs structuraux A) porte(nt) une ou plusieurs chaînes latérales hydrophobes.

4. Mélange selon l'une quelconque des revendications 1 à 3, **caractérisé en ce que** les chaînes latérales formées à partir des motifs structuraux X sont des séquences polyoxyéthylène ou polyoxyéthylène/polyoxypropylène hydrophiles.

5. Mélange selon la revendication 4, **caractérisé en ce que** le monomère constituant les motifs structuraux X (les monomères constituant le motif structural X) est (sont) un mélange d'oxyde d'éthylène et d'oxyde de propylène, ayant de préférence une teneur en oxyde de propylène de 5 à 20 %.

6. Mélange selon la revendication 4 ou 5, **caractérisé en ce que** la totalité ou une partie des chaînes latérales formées à partir des séquences oxyde d'éthylène ou oxyde d'éthylène/oxyde de propylène hydrophiles se terminent chacune par une séquence hydrophobe de préférence par un poly- ou oligo-oxyalkylène hydrophobe ou par une chaîne alkyle en $C_{10}$ à $C_{30}$ ramifiée ou non ramifiée.

7. Mélange selon l'une quelconque des revendications 1 à 6, **caractérisé en ce que** le monomère constituant le(s) motif(s) structural(raux) A' est l'anhydride maléique et le monomère constituant les motifs structuraux X est l'oxyde d'éthylène.

8. Mélange selon l'une quelconque des revendications 1 à 6, **caractérisé en ce que** le monomère constituant le(s) motif(s) structural(raux) A' est l'alcool vinylique et le monomère constituant les motifs structural X est l'acide (méth) acrylique ou l'oxyde d'éthylène ou un mélange d'oxyde d'éthylène et d'oxyde de propylène.

9. Utilisation d'un mélange selon l'une quelconque des revendications 1 à 8, pour la stabilisation d'émulsions, en particulier d'émulsions huile/eau, d'émulsions eau/huile, de microémulsions ou d'émulsions multiples telles que des émulsions huile/eau/huile ou des émulsions eau/huile/eau.

10. Microémulsion, contenant un tensioactif et un co-tensioactif, **caractérisée en ce qu'**on utilise comme co-tensioactif une substance ou un mélange de substances correspondant à la définition dans l'une quelconque des revendications 1 à 8.

11. Utilisation d'un mélange selon l'une quelconque des revendications 1 à 8 ou d'une microémulsion selon la revendication 10, en tant que produit de lavage, émulsifiant, régulateur de mousse, agent mouillant pour surfaces dures ou en tant que milieu réactionnel pour des réactions organiques, inorganiques, bio-organiques ou photochimiques.

12. Utilisation selon la revendication 11, dans des produits lessiviels, des compositions de tensioactifs pour le nettoyage de surfaces dures, des humectants, des compositions cosmétiques, pharmaceutiques et phytosanitaires, des peintures, des produits de revêtement, des adhésifs, des dégraissants pour le cuir, des compositions pour l'industrie textile, la transformation de fibres, la transformation de métaux, l'industrie alimentaire, le traitement de l'eau, l'industrie du papier, la fermentation, la transformation de minéraux, l'ignifugation ou dans des polymérisations en émulsion.

13. Produits de lavage, de nettoyage, désinfectants, agents mouillants, produits de revêtement, adhésifs, dégraissants pour le cuir, humectants ou produits de traitement de textiles ou composition pharmaceutique, de produit alimentaire, phytosanitaire ou cosmétique, en particulier produits de protection solaire, pour le soin de la peau ou de coiffage, gels de douche, shampooings, additifs pour le bain ou huiles essentielles, contenant, outre des composants usuels, un mélange selon l'une quelconque des revendications 1 à 8 ou une microémulsion selon la revendication 10.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19839054 A
- DE 19634477 A
- EP 0794245 A
- EP 0412389 A
- DE 10243363 A
- DE 10243361 A
- DE 10243360 A
- DE 10243365 A
- DE 10243366 A
- DE 10243362 A
- DE 4325237 A
- DE 10118480 A
- DE 10233740
- DE 102004007885

### In der Beschreibung aufgeführte Nicht-Patentliteratur

- **H.-F. Eicke.** Mikroemulsionen - eine wissenschaftliche und anwendungstechnische Fundgrube?. *SÖFW-Journal,* 1992, vol. 118, 311-314
- Kosmetische Färbemittel. Farbstoffkomission der Deutschen Forschungsgemeinschaft. Verlag Chemie, 1984